(19) **Europäisches Patentamt / European Patent Office / Office européen des brevets**

(11) **EP 3 900 814 B1**

(12) # EUROPEAN PATENT SPECIFICATION

(45) Date of publication and mention
of the grant of the patent:
**30.07.2025 Bulletin 2025/31**

(21) Application number: **20171199.1**

(22) Date of filing: **23.04.2020**

(51) International Patent Classification (IPC):
**B01D 63/02** *(2006.01)* **A61M 1/36** *(2006.01)*

(52) Cooperative Patent Classification (CPC):
**B01D 63/02; A61M 1/3643; A61M 1/3673;
A61M 2206/20; B01D 2313/10; B01D 2313/21**

(54) **DIALYZER HEADER CAP**

DIALYSATORKOPFKAPPE

CAPUCHON DE COLLECTEUR DE DIALYSEUR

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**27.10.2021 Bulletin 2021/43**

(73) Proprietor: **Bellco S.r.l.
41037 Mirandola (IT)**

(72) Inventor: **MAROTTA, Gaspare
41037 Modena (IT)**

(74) Representative: **Maschio & Soames IP Ltd
30 Carlton Crescent
Southampton SO15 2EW (GB)**

(56) References cited:
| | |
|---|---|
| **EP-A1- 1 837 046** | **WO-A1-2005/044428** |
| **WO-A1-2011/032154** | **JP-A- 2003 154 237** |
| **JP-A- 2005 270 289** | **US-A- 4 141 835** |
| **US-A1- 2005 247 618** | **US-A1- 2019 209 767** |

**Description**

**FIELD**

The present invention relates to apparatus for use in hemodialysis, and in particular header caps for a dialyzer.

**BACKGROUND**

A dialyzer is a type of filter used in hemodialysis. A typical dialyzer comprises two main units. Firstly, a global housing section and secondly a membrane bundle. The membrane bundle sits inside the housing and comprises a large number of semi-permeable hollow fibres, each with a diameter of around 200 microns.

During hemodialysis, at high pressure blood from a patient enters the hollow fibres of a dialyzer at one end. Dialysis solution or dialysate passes through the dialyzer in the opposite direction to the blood, outside of the fibres. The dialysate comprises an aqueous electrolyte solution, and the exact composition varies depending on the patient to be treated. Low molecular weight metabolites, such as urea, pass from the blood into the dialysate through osmotic action.

The hemodialysis process aims to correct the electrolyte and acid-base imbalance in the blood, which is symptomatic of some diseases, and in particular renal failure.

Blood enters a dialyzer at one end of the dialyzer housing. Known dialyzers comprise an inlet port which is directly centrally aligned along the longitudinal axis of the dialyzer. The change in blood flow distribution and pressure differentials created as the blood enters the dialyzer may cause mechanical stress to the blood and blood cells. Blood flow efficiency is also potentially impaired by the change in pressure, container volume and shape, such as sudden warps or restrictions, with varying fluid pressures as the blood passed from the inlet port into the hollow fibres. Furthermore, efficient mass osmotic transfer requires a homogenous distribution within each individual hollow fibre, as well as between the fibres, which is also subject to pressure differentials created by incoming blood flow.

The dialyzer mass transfer-area coefficient (KoA) for an area is a determinant of urea removal during hemodialysis, and is considered to be constant for a given dialyzer.

Increasing the koA of a dialyzer, without increasing costs or time needed for a dialysis session, may improve patient outcomes due to the improved efficiencies of the Document US4141835 discloses a dialyser head according to the preamble of claim 1.

In view of the above, there is a need for an improved dialyzer to improve the blood flow within the device and overall osmotic transfer efficiency within the dialyzer fibers.

**SUMMARY**

The present inventors have determined that the structure of the header cap of the dialyzer can play a significant role in determining how blood flow in the dialyser is managed. The invention is disclosed in the appended set of claims.

In one embodiment, the dialyzer header cap of the invention improves uniformity of blood distribution to the hollow fibres of the dialyzer.

In one embodiment, the geometry of the dialyzer header cap is configured to reduce mechanical stress to blood flow within it, compared to existing designs, such as that shown in **Figure 1.** Preferably mechanical stress is reduced by 5% or more, 10% or more, 20% or more or 50% or more. In a most preferable embodiment, mechanical stress is reduced by 12% or more.

In one embodiment, the present invention includes a dialyzer header cap wherein blood transfer efficiency into the hollow fibres of the dialyzer is improved by at least 5%, preferably at least 10%, preferably at least 20%, preferably at least 30%, preferably at least 35%, preferably at least 40%. In a most preferable embodiment, blood transfer efficiency into the hollow fibres of the dialyzer is improved by around 30-35%, most preferably by around 33% or about 33%.

In one embodiment, the present invention includes a dialyzer header cap wherein mass transfer efficiency of urea is increased by up to 5% of urea koA, preferably up to 10% of urea koA, more preferably up to 20% of urea koA and more preferably up to 30% of urea koA.

In one embodiment, the geometry of the dialyzer header cap is configured to reduce air bubble formation during the priming phase. Preferably, said air bubble formation is reduced by at least 10%, preferably by at least 20 %, preferably by at least 30%, most preferably by at least 40%. In a preferred embodiment, air bubble formation is reduced by around 30-35%, most preferably by around 33%.

In one embodiment, the dialyzer header cap is configured to increase uniformity of blood flow distribution at the outlet. Preferably, blood flow distribution uniformity is increased by at least 10%, at least 20 %, at least 30%, at least 40%, at least 50% or most preferably at least 70%.

The dialyzer header cap described herein has an asymmetrical shape. The dialyzer header cap of the invention may be concave. Preferably, the header cap of the invention is both concave and asymmetrical.

The dialyzer header cap shape can in some embodiments be defined by (a) asymmetrical concave shape, and (b) by an internal volume generated by the cross-section outlet along a rotated axial direction less than 45 degrees, preferably in a range between 20 and 45 degrees. In some embodiments the dialyzer header cap shape can be defined by the inlet to the header cap being formed of a channel tangential to the upper curvature of the header cap. Preferably, in this header cap shape, the inlet port of said header cap is essentially orthogonal to the outlet port.

In one embodiment, the dialyzer header cap shape can be defined wherein the internal profile can be described by a fourth-degree polynomial surface in three dimensions as indicated in [Equation 1] with mixed coefficients as indicated in Table 1. In one embodiment, the dialyzer internal header cap shape can be defined by [Equation 1] and as shown in Figure 8a or with a down-scale reduction along Z direction up to 50% as shown in Figure 8b.

$$Z = f(x, y) = p00 + p10\,x + p01\,y + p20\,x^2 + p11\,x\,y + p02\,y^2 + p30\,x^3 + p21\,x^2 y$$
$$+ p12\,x\,y^2 + p03\,y^3 + p40\,x^4 + p31\,x^3 y + p22\,x^2 y^2 + p13\,x\,y^3 + p04\,y^4$$

[Equation 1]

Equation 1 may describe the internal of the header cap of the invention with constant coefficients for the shape with maximum angle rotation of 45 degrees to the vertical. If we desire to obtain the shape with the minimum angle rotation of 20 degrees to the vertical we need to scale down the Z value only (not the constant coefficient in Table 1) obtained from the poly equation with a Z scale-down factor.

In one embodiment, the dialyzer header cap shape comprises an internal curved surface. In particular, in one embodiment the dialyzer header cap is in the shape of an asymmetrical elliptic paraboloid. Preferably the internal curved surface forms a hollow with a wide opening or wide open section which comprises the outlet port. The inlet port is attached to the upper curved section. Said inlet port being orthogonal to or essentially orthogonal to the outlet port. The curved surface is asymmetrical, and most preferably the inlet port is located on the internal curved surface at or around the most distal point from the outlet port. While the internal surface is curved, the external surface may be of any shape, but it is envisaged in most embodiments, such as those shown in the enclosed figures, that the external surface of the header cap described herein will be shaped to reflect the internal curvature. Wherein we refer to the "shape"" of the header cap of the invention herein, we are in general referring to the internal shape, which affects blood flow within the device, unless it is stated otherwise.

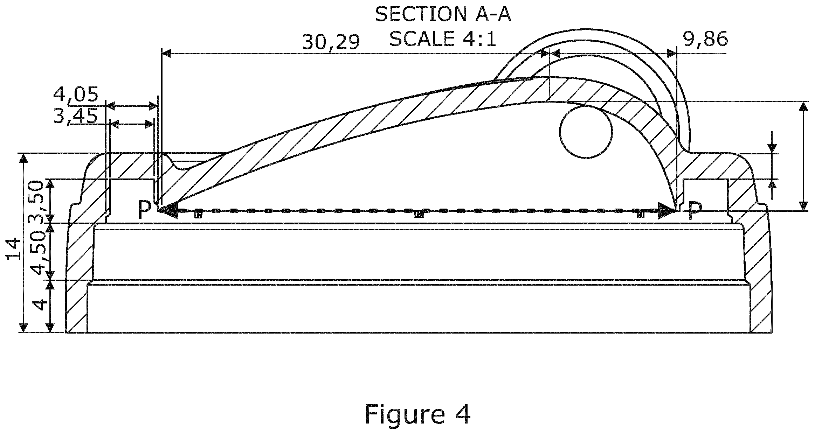

A header cap of the current invention could be considered as half of a hollow sphere or spheroid, wherein the open widest part of the hollow sphere is the outlet port, and wherein an inlet port is attached essentially orthogonally to the side of the sphere. Most preferably the dialyzer header cap is not an even half-spherical shape but rather is an asymmetrical shape (as shown for example in **Figure 4).**

The shape of the dialyzer header cap is described as a hollow halved solid of revolution, wherein the inlet port is essentially orthogonal to the outlet.

The outlet in this embodiment being in the plane of revolution, and the full width of the hollow.

The shape of a preferred embodiment of the dialyzer header cap may be described as or essentially as an elliptic paraboloid of revolution without symmetry, wherein the inlet port is essentially orthogonal to the outlet and essentially positioned in the cusp domain. The outlet in this embodiment being in the plane of revolution, and preferably the full width of the hollow.

The dialyzer header cap shape can in some embodiments be defined by (a) asymmetrical concave shape, and (b) the top inflection section is radially shifted over the range of 0.4-0.6 times the header cap outlet radius and (c) axially inclined over the range of 45-60 degrees respect to the central axis. Said shape may be described by Equation 1, as recited herein.

The dialyzer header cap shape can in some embodiments be defined as comprising

    (a) an asymmetrical internal concave shape essentially similar to an elliptic paraboloid without symmetry;

    (b) a top cusp section which is radially shifted over the range of 0.4-0.6 times the header cap outlet internal radius; and

    (c) an axial incline over the range of 20-45 degrees with respect to the central axis.

The dialyzer header cap described herein may be formed of a plastic, a plastic polycarbonate, polypropylene, a rubber, or a composite. Preferably the header cap is made of a plastic polycarbonate or polypropylene material.

The dialyzer header cap described herein may comprise a plug fit, screw fit, stretch fit, Luer-Lock or snap fit attachment, at the outlet, inlet or both.

A dialyzer header cap described herein may be fully or partially coated with an antimicrobial agent, an antibacterial agent, a disinfectant, a lubricant, an anti-coagulant, an anti-viral agent or any combination of these.

Also described herein is a dialyzer comprising the dialyzer header cap of the invention. The header cap of the invention is fitted to one end of the dialyzer housing. Preferably the header cap may screw fit, snap fit, plug fit or stretch fit over the end of the dialyzer housing in order to attach. In preferred embodiments, the output port is the part of the header cap which attaches to the dialyzer. Preferably the entire width of the outlet port of the header cap fits over, within or to the dialyzer body.

The current invention also encompasses a dialysis kit comprising a dialyzer header cap described herein. Said kit may further include a main housing containing a bundle of hollow fibres.

Use of a dialyzer header cap is also described herein. Preferably the header cap may be used in in priming a dialyzer. The header cap of the invention reduces air bubble formation during the priming process, and thus increases priming efficiency. In a preferred embodiment, priming efficiency is increased by up to 10%, up to 20%, up to 30% or up to 40%. In a most preferred embodiment, priming efficiency is increased by 25-30%, most preferably 25-30%, most preferably by around 29-30% or around 29.5%.

The advantages of the dialyzer header cap as disclosed herein include overall improved dialysis efficiency. This arises from the more efficient mass transfer of urea and other solutes dissolved in the blood due to the fluid distribution in the dialyzer being spread more evenly across the bundle of hollow fibres.

Less mechanical damage occurs to the blood cells when the improved dialyzer header cap is used. This helps to prevent reduced blood oxygen transportation and increased clotting and/or blood viscosity

Priming is more efficient using the improved header cap of the invention. Air bubble formation, and the potentially serious side effects of air embolisms in a patient, is thus reduced.

Overall, the aim of the header cap of the current invention is to improve patient outcomes.

**FIGURE LEGEND**

Figure 1 shows the current geometry of dialyzer header caps, comprising a narrow inlet port and a wider outlet port, wherein both ports are directly centrally aligned along the same axis. Blood (or any fluid) enters the inlet port in the approximate direction shown by the narrow arrow. Blood leaves the header cap through the full width of the base, in the direction indicated approximately by the board arrow.

Figure 2 shows a diagram of blood flow through a dialyzer, illustrating the change in blood flow distribution profile using the dialyzer header cap of figure 1 compared to a dialyzer header cap of the invention as shown in Figure 3.

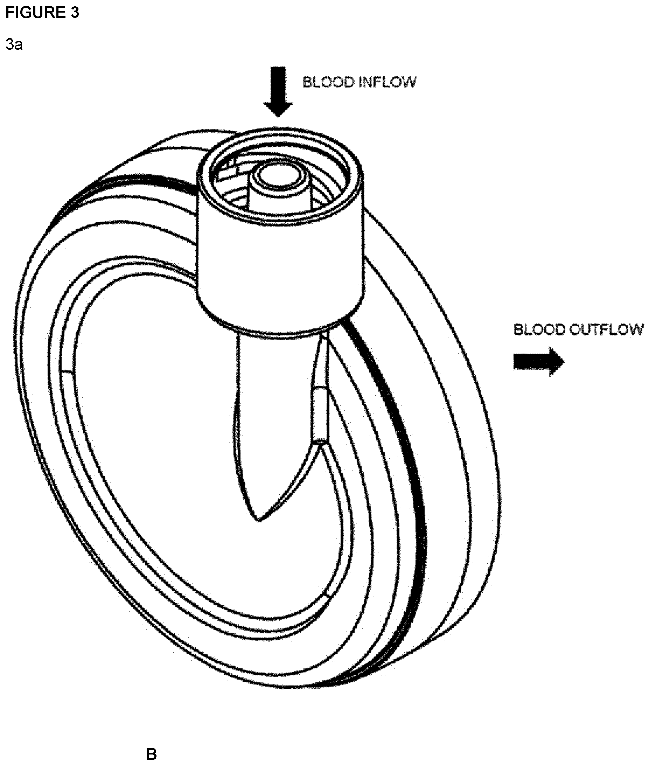

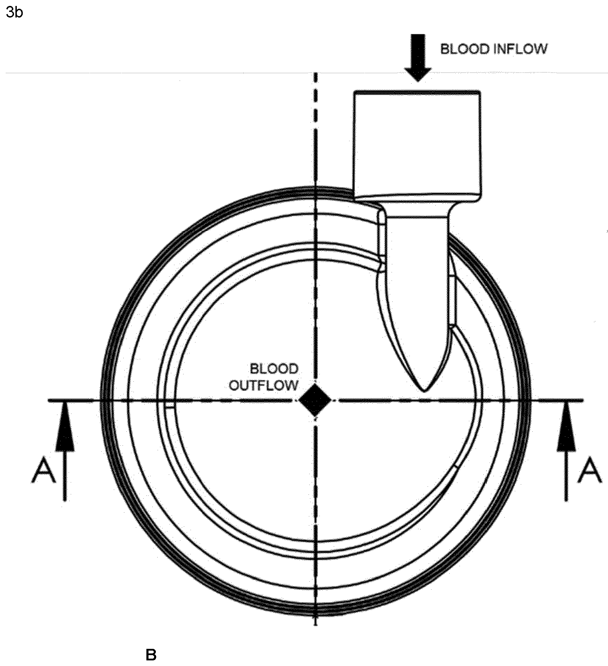

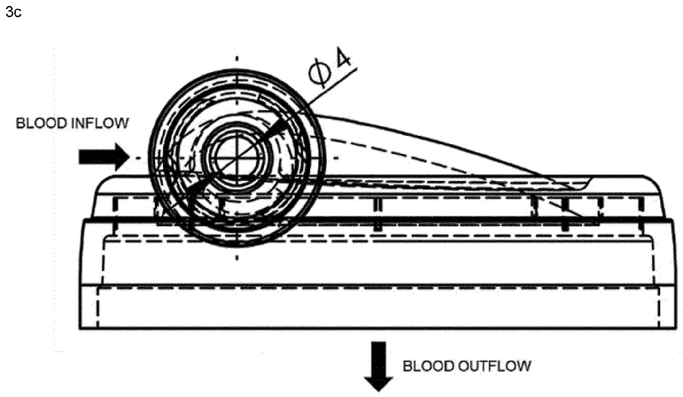

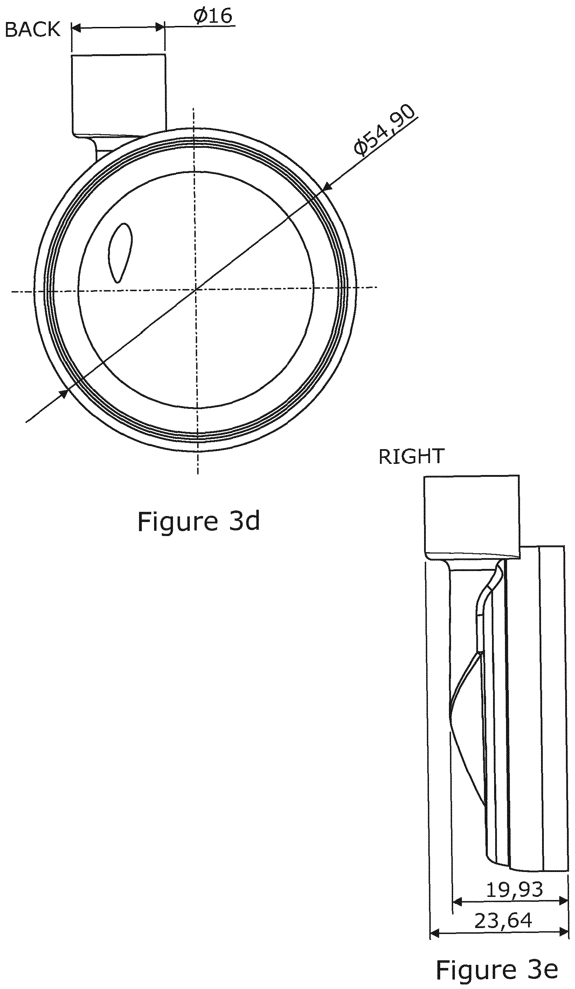

Figure 3 shows a series of views of an example of the dialyzer header cap of the invention. The top panel, Figure 3a, shows a side view showing the inlet port facing upwards. Blood (or any fluid) enters in the direction pf the arrow marked "blood inflow". The inside of the header cap is hollow (as shown in Figure 3c cross-section). The outlet for the blood from the header cap includes the full width of the base of the device, and blood flows in the direction approximately indicated by the arrow marked blood outflow. The second panel, 3b, shows the same header cap of 3a viewed from above. The third panel, 3c, shows the header cap in cross section along the line A-A . Visible are the details of a standard female Luer Lock. The fourth panel, Figure 3d, shows Figure 3b turned upside down. The fifth panel, Figure 3e shows a right hand side view of the same embodiment shown in panels 3a-3d. The panels of Figure 3 show some preferred maximum lengths of various dimensions of the header cap, such as the maximum and minimum external height of the header cap, the external diameter size of the Luer lock section and the maximum preferred external diameter sizer of the outlet header cap section. All dimensions are given in millimetres (mm).

Figure 4 shows a cross-section of the embodiment of **Figure 3** cut through the line A-A in the first panel of **Figure 3b,** looking in the opposite direction to **Figure 3c.** Dimensions given in millimetres (mm).

Figure 5 shows a cross-section of the embodiment of **Figure 3b** cut through the line B-B. Dimensions given in millimetres (mm).

Figure 6a shows a pictorial representation of the blood flow velocity through the inlet port (named "inflow blood port" in **Figure 6a)** of a dialyzer header cap of the invention across the width of the dialyzer, compared to dialyzer header caps of the prior art (shown in figure 1).

Figure 6b shows a pictorial representation of the blood flow velocity through the outlet port (named "Outflow blood port" in **Figure 6b)** of a dialyzer header cap of the invention across the width of the dialyzer, compared to dialyzer header caps of the prior art (shown in figure 1). The term "Qb" in this figure refers to volumetric blood flow rate.

**Figure 7** shows the difference in trapped air volume during the priming phase in a dialysis flow path using a dialyzer header cap of the prior art (top panel **7a)** compared to a header cap of the present invention (lower panel **7b).** The black points represent the quantity of the air volume fraction in the header cap. The white circles represent instead the fluid domain without air trapped (i.e. air volume fraction = (volume of air/ (volume of air+volume of water))

**Figure 8** shows the surface of the internal section of the dialyzer header cap when developed along the Z direction in upper panel **Figure 8a** and with a down-scale reduction along Z direction up to 50% as shown in lower panel **Figure 8b.**

## DETAILED DESCRIPTION

### Definitions

Unless defined otherwise, all technical and scientific terms used generally have the same meaning as commonly understood by one of ordinary skill in the art.

The articles "a" and "an" are used to refer to one or to over one (*i.e.*, to at least one) of the grammatical object of the article. For example, "an element" means one element or over one element.

The term "approximately" as used herein means not exactly limited to. In particular, when used in relation to a direction, "approximately" means the direction may vary by several degrees to the central axis. For example, the direction of blood flow (or any fluid) shown in Figures 1-3 clearly must flow mainly in one direction, but due to the width of the header cap, and flows and eddies in the fluid, there will be some variation in the direction of flow, hence the description "approximately".

The term "asymmetrical" refers to a feature which lacks symmetry in at least one plane, preferably all planes. That is, the feature cannot be found identical each side of a central line.

The term "asymmetric elliptic paraboloid" refers to the shape of a surface defined by Equation 1 which particularly defines the flooded surface by the blood flow inside the header cap.

The term "cusp domain" refers to the farthest section of the asymmetric elliptic paraboloid where it will be connected the inlet section orthogonal to the header cap outlet.

The terms "blood flow homogenization" and "blood flow distribution uniformity" are used interchangeably and refer to the dispersal of the blood throughout the vessel in which it flows, wherein said vessel is preferably hollow tubing or pipes. In other words, blood flow homogenization gives a measure of how equally blood flows equally throughout the vessel or if blood speed and volume of flow is concentrated in specific areas or section of the vessel, especially wherein said vessel is a dialyzer header cap. Blood flow homogenization can be measured using computational fluid dynamic (CFD) simulation for example. Blood flow which is more uniform or has increased homogenization means it is more evening distributed throughout the vessel it is flowing through. This can be expressed as a relative percentage increase or decrease of blood flow uniformity or blood flow homogenization.

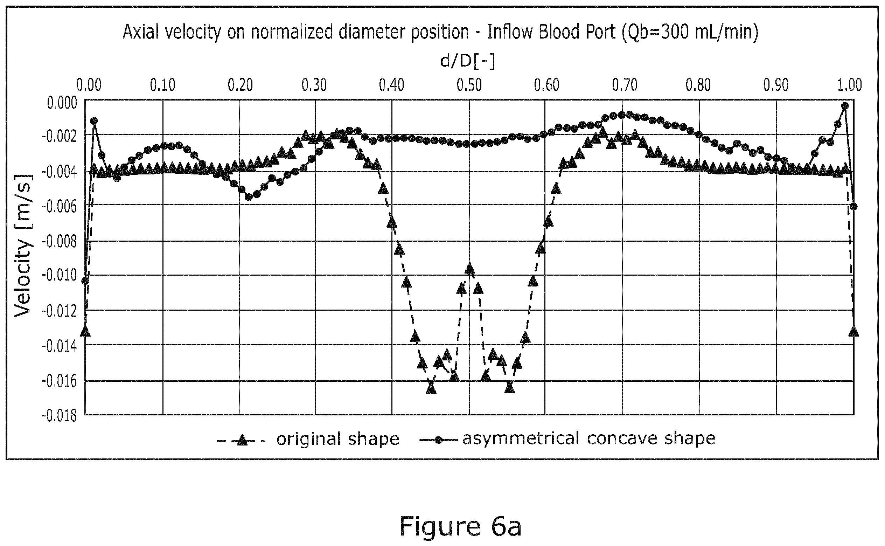

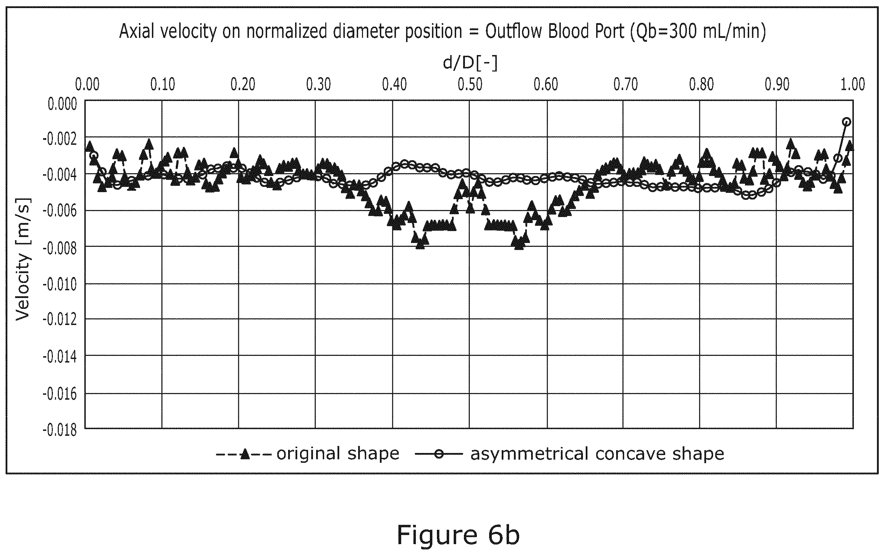

The term "blood transfer efficiency" as used herein refers to the amount and speed of blood transfer from one container or other part of a dialysis flow path to another. In particular it refers to the transfer of blood from connecting tubing, through a dialyzer header cap and into the hollow fibres of a dialyzer. Blood transfer efficiency can be measured by a comparison between an inflow velocity profile of the header cap of the invention (see **Figure 6),** compared to an inflow velocity profile of known header caps, such as that shown in **Figure 1.** Efficient blood transfer results in quick movement of the blood through a dialysis flow.

The term "collapsible" refers to a structure or feature which can be folded into a more compact shape.

The term "compressible" refers to a structure or feature which can be flattened across one dimension, resulting in reduced size, in particular reduced height or reduced width.

The term "comprising" includes, but is not limited to, whatever follows the word "comprising." Use of the term indicates the listed elements are required or mandatory but that other elements are optional and may be present.

The term "consisting of" includes and is limited to whatever follows the phrase "consisting of." The phrase indicates the limited elements are required or mandatory and that no other elements may be present.

The term "consisting essentially of" includes whatever follows the term "consisting essentially of" and additional elements, structures, acts or features that do not affect the basic operation of the apparatus, structure or method described.

The term "concave" or "concave shape" means a feature having an outline or surface that curves inwards like the interior of a circle or sphere.

The term "dialysate" describes a fluid into or out of which solutes from a fluid to be dialyzed diffuse through a membrane. An initial dialysate used for therapy typically contains electrolytes close in concentration to the physiological concentration of electrolytes found in blood. However, the concentration of the dialysate can change over the course of therapy, and can further be adjusted as desired.

The term "dialysis flow path" refers to a fluid pathway or passageway configured to convey a fluid, such as dialysate and/or blood, wherein said pathway forms at least part of, preferably the whole of, a fluid circuit for peritoneal dialysis, hemodialysis, hemofiltration, hemodiafiltration or ultrafiltration. A dialysis machine may be included within the flow path. A dialyzer may be included in the flow path.

The term "dialyzer" refers to a cartridge or container with two flow paths separated by semi-permeable membranes. One flow path is for blood and one flow path is for dialysate. The term "dialyzer" can refer to a cartridge or container with two flow paths separated by semi-permeable membranes. The membranes are preferably hollow fibres, but can also be in for form of flat sheets, or spiral wound or other conventional forms known to those of skill in the art. The term "hollow fibres" is used throughout, but can be interchanged with any other shape of membrane. Commonly the membranes form a central bundle. Membranes can be selected from any one or combination of materials: polysulfone, polyethersulfone, poly (methyl methacrylate), modified cellulose, or other materials known to those skilled in the art. The current invention is concerned with a dialyzer header cap which forms the entry into the dialyzer for the blood in the blood flow path.

The term "dialyzer header cap" (or simply "header cap" or "dialyzer cap", as used interchangeably herein) as used herein refers to entry port into a dialyzer for fluid, preferably blood. A dialyzer header cap directs fluid in a flow path into a dialyzer. More specifically, said header cap fits (using some form of connection, such as snap fit, stretch fit or screw fit) into the top of the body of a dialyzer and forms and entry port, wherein blood from the flow path enters the header cap at an inlet port and exits the header cap at an outlet port into the hollow fibres of the dialyzer.

The terms "dialyzer mass transfer-area coefficient for urea"," mass transfer efficiency of urea", "urea mass transfer efficiency" or "Urea koA" all have the same meaning and refer to a determinant of urea removal during hemodialysis. KoA, is a measure of dialyzer efficiency in clearing urea and solutes of similar molecular weight. The KoA is the maximum theoretical clearance of the dialyzer in millilitres per minute for a given solute at infinite blood and dialysate flow rates Urea KoA values usually vary between around 500 to 1200 ml/min depending on the dialyzer model. Urea KoA is closely linked to the dialyzer design. In particular, the teachings of Kim et al, Blood Purification, Vol 28(3); pp260-7, 2009, "Effects of Arterial Port Design on Blood Flow Distribution in Hemodialyzers" and "Kulz et al, Nephrol. Dial. Transplant. Vol 17(8), pp1475-9, 2002, "In vitro and in vivo evaluation of a new dialyzer" were used to compare blood flow homogenization using different shape of dialyzer header cap and thus indirectly estimate Urea KoA.

The term "fluid" can be any substance without a fixed shape that yields easily to external pressure such as a gas or a liquid. Specifically, the fluid can be water containing any solutes at any concentration. The fluid can also be dialysate of any type including fresh, partially used, or spent. The fluid can also contain one or more dissolved gas. The fluid may be blood.

The term "formed" or "formed of" as used herein refers to a material comprising any of the materials, elements, composited or compounds listed.

The term "fourth-degree polynomial surface equation" also known as " quartic plane curve" refers to a surface developed in the three-dimensional domain described by:

$$Z = f(x,y) = p00 + p10\,x + p01\,y + p20\,x^2 + p11\,x\,y + p02\,y^2 + p30\,x^3 + p21\,x^2 y$$
[Equation 1]
$$+ p12\,x\,y^2 + p03\,y^3 + p40\,x^4 + p31\,x^3 y + p22\,x^2 y^2 + p13\,x\,y^3 + p04\,y^4$$

Fourth-degree polynomial surface equations have these characteristics:

- Four connected components
- Twenty-eight bi-tangents
- Three ordinary double points.
- 15 constant coefficients
- 14 degrees of freedom

Preferably in the current invention, the surface of the internal section of the dialyzer header cap can be described by a fourth order polynomial equation wherein the coefficients are shown in the Table 1 where to each value it is referred also to the bounds of application in the space domain. The surface of the internal section of the dialyzer header cap can be developed along the Z direction as shown in Figure 8a with a down-scale reduction along Z direction up to 50% as shown in Figure 8b.

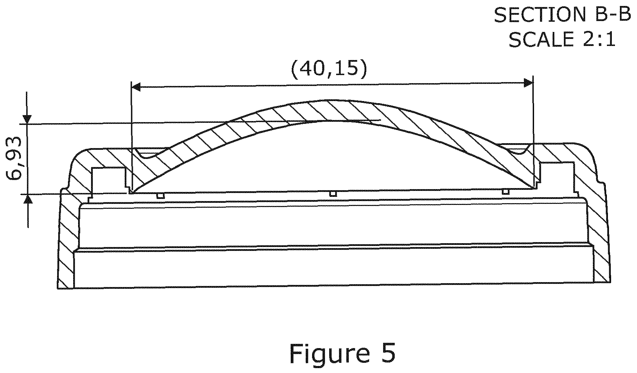

The term "hollow" as used herein means having an empty space inside. A feature which is hollow, such as the hollow fibres of the dialyzer, has an internal volume. The inside of the header cap described herein is referred herein as having a "hollow space" or "hollow chamber". This indicates the space inside the header cap which blood fills on entry. This is shown in at least figures 4 and 5.

The term "inflow" refers to the direction or position of a fluid flowing into a vessel such as a tube or a dialyzer header cap of the invention. In the figures the inflow direction is indicated by an inward pointing arrow.

The term "inlet" can refer to a portion of a component through which fluid and/or gas can be drawn into a component, conduit, or fluid passageway of any type.

The term "inlet port" (or "input port", which can be used interchangeably) refers to an inlet of a dialyzer header cap for fluid, commonly blood, to enter the dialyzer header cap.

The term "measuring" or "to measure" can refer to determining any parameter or variable. The parameter or variable can relate to any state or value of a system, component, fluid, gas, or mixtures of one or more gases or fluids.

The term "measurement" refers to data or information of the parameter or variable determined.

The term "mechanical stress" refers to the internal forces that arise in a body being deformed as a result of external forces. In particular herein, the term mechanical stress refers to the stress experienced by blood, most specifically cells in blood (red blood cells, white blood cells and others) when undergoing dialysis. Such mechanical stress occurs normally during blood circulation, but may be greatly increased when coming into contact with devices found in a dialysis flow path, such as a dialysis machine and dialyzer, and in particular with the walls of said devices. Mechanical stress of blood can produce adverse effects such as a reduction in oxygen circulation, increased or reduced ability to clot, increased or reduced viscosity, altered electrolyte balance in the blood and reduced immune system function. Mechanical stress in blood can be measured by several experimental measurements, such as the haemolysis index. The haemolysis index is a measurement of the colour of blood plasma, a darker colour being indicative of more rupturing of red blood cells and thus mechanical stress. The dialyzer header cap of the current invention shows reduced mechanical stress relating to impact with the walls of the header cap when flowing through the inlet to the header cap.

The term "orthogonal" refers to a feature or axis at right angles to another feature or axis. Wherein "essentially orthogonal" is used, this term encompasses orthogonal and slightly off axis, such as 90-95 degrees in relation to an axis, 90-100 degrees in relation to an axis or 90-105 degrees in relation to an axis. The terms "essentially orthogonal" and "essentially 90 degrees" also include a feature 85-90 degrees to an axis, 80-90 degrees to an axis or 75-90 degrees to an axis. The term "perpendicular" can be used interchangeably with the term "orthogonal". The term "essentially perpendicular" can be used interchangeably with "essentially orthogonal".

The term "outflow" refers to the direction or position of a fluid flowing out of a vessel such as a tube or a dialyzer header cap of the invention. In the figures the outflow direction is indicated by an outward pointing arrow.

The term "outlet" refers to a portion of a component through which fluid or gas can be pulled out of a component, conduit, or fluid passageway of any type.

The term "outlet port" refers to an outlet of the dialyzer header cap for fluid, commonly blood, to leave the dialyzer header cap. In a preferred embodiment, the outlet port of the header cap of the invention includes the entire width, or almost the entire width, of the base of the header cap. An outlet port may be "essentially as wide" as the dialyzer. That is, the outlet port is so wide that a fluid flowing through it is not constricted or very little constriction occurs when flowing into the dialyzer, because the outlet port is very similar width to the dialyzer. An outlet port may be interchangeably referred to as an "outlet". The "initial portion" of the outlet port refers to the portion most distant to the body of the dialyzer header cap. The terms "outlet port" and "blood outlet port" are used interchangeably.

The term "pressure" refers to a force exerted by a gas or liquid on the walls of a component, container, or conduit.

The term "priming" or "priming phase" refers to the process of removing air from the tubing or dialyzer by allowing sterile priming solution, usually a saline solution, to flow through it. Insufficient priming can cause problems such as foam forming in the circulated blood during use, and clotting forming on the hollow fibres in the dialyzer.

The term "rigid" as used herein refers to a material which is unable are barely able to bend or be forced out of shape. A material which is not flexible.

The term "solid of revolution" as used herein refers to is a solid figure obtained by rotating a plane curve 360° around an axis of revolution that lies on the same plane. Half a solid of revolution is created by rotating a plane curve 180° around an axis of revolution that lies on the same plane. The solid of revolutions and half solids of revolution described herein may be hollow.

The term "tangent" as used herein means a straight line or plane that touches a curve or curved surface at a point. The term "tangential" means relating to or along a tangent.

The term "tear drop shape" or "tear drop shaped" refers to something shaped like a falling drop of thin liquid, having a globular form at one end and tapering to a point at the opposite end. This can be a three dimensional shape. In naturally forming tears of liquid, the globular end is the lowest end due to gravity. The term "lachrymifom" is used interchangeably herein to mean "tear drop shape".

## Apparatus

The current geometry of dialyzer header caps is shown in **Figure 1.** Such header caps comprise a narrow inlet port and a wider outlet port, wherein both ports are directly centrally aligned along the same axis.

**Figure 2** shows a schematic diagram of an entire dialyzer wherein during use blood enters through the blood inlet at the top, in the direction indicated by the arrow. The blood passes through a header cap (not shown) and enters a bundle of semi-permeable hollow fibres in the position represented by the large central rectangle of **Figure 2.** The velocity profile of the blood entering the hollow fibres through the header cap or **Figure 1** is shown as a steeply angled curve. A flow profile such as this leads to a high blood distribution to the central section of the hollow fibre bundle only. This reduces the transfer efficiency of urea and other electrolytes into the dialysate.

**Figure 2** also shows the flow profile of the improved dialyzer header cap described herein. This is a much more even profile, showing that the blood flow enters the hollow fibres in a much more even spread across the dialyzer.

Fluid flows into the dialyzer header cap through the inlet port and may fill the entire hollow inside of the header cap. The indications of size, given herein on **Figures 3-5,** are not limiting, and it is envisaged that the header cap could be made of any size in order to fit any size of dialyzer.

In a preferred embodiment, the header cap of the current invention has a concave shape

In another preferred embodiment, the header cap of the current invention has an asymmetrical concave shape.

In a preferred embodiment, the header cap described herein has an internal volume generated by the cross-section of the outlet along a rotated axial direction of between 20 and 45 degrees.

Most preferably along a rotated axial direction less than about 40 degrees, less than about 35 degrees or less than about 30 degrees.

In a preferred embodiment, the outlet of the header cap of the invention is circular (as shown in **Figure 3a-e,** or oval. In a most preferred embodiment, the position of the outlet of the header cap can be described as located along a tangential line that bisects the outlet at between 20 and 45 degrees, most preferably at 40-45 degrees, most preferably at 35-45 degrees. Said tangential line preferably does not bisect the outlet centrally.

As shown in **Figures 3, 4 and 5,** the dialyzer header cap may also comprise a base section. Said base section is adjacent to the outlet, that is the section where the blood is flowing into each hollow fibers of the dialyzer, in the direction of the arrows indicated and labelled "Blood Outflow". The base section may preferably comprise an extension of the material of the outlet. Said base section may engage with or fit into or around the housing of a dialyzer. Attachment of the base section to the dialyzer may be screw fit, snap fit, stretch fit (wherein the open outlet stretches around the dialyzer to encompass it) or comprise another attachment mechanism. In the absence of a base section, the outlet may engage with or fit into or around the housing of a dialyzer in the same way. The lateral section of the base of header cap is welded to the header cap, preferably using an ultrasonic method.

The header cap of the invention may be made from a rigid or flexible material. Said material may be a plastic, a rubber, or a composite. Preferably the header cap is made of a plastic polycarbonate or polypropylene material.

The header cap of the invention has a thickness (shown in **Figure 4** as hatched lines). The thickness of the header cap depends upon the material from which it is made, the size of the header cap and how the header cap attaches to the dialyzer.

The inlet port of the header cap may comprise one or more attachment pieces, as shown in **Figures 3-5** either formed contiguously with the header cap, or attachable thereto. Said attachment pieces allow the attachment of other components of a dialysis flow path, such as tubing, to the inlet port.

Such attachment pieces may be screw fit, snap fit, stretch fit or comprise another attachment mechanism.

The header cap described herein should not be collapsible. The header cap should be formed of a rigid or essentially rigid material. Rigidity is necessary in order to avoid enlargement of blood stagnation before and after the hollow fiber section of the dialyzer.

The shape of a preferred embodiment of the dialyzer header cap may be described as a hollow halved solid of revolution. That is, a hollow half a solid of revolution created by rotating a plane curve 180° around an axis of revolution that lies on the same plane. Preferably the plane curve is asymmetrical. That is wherein the plane curve is not a normal curve.

The dialyzer header cap of the invention is a hollow half a solid of revolution having a lachrymiform, tear drop shape or asymmetric elliptic paraboloid. Wherein the outlet of the header cap is formed along the axis of revolution, and wherein the inlet is attached on or around the most distal point of the header cap from the outlet port. In other words, the inlet is attached at the widest point of the half teardrop shape. Preferably the inlet is essentially orthogonal to the vertical. Such an embodiment is shown in **Figure 4** for example.

In **Figure 4,** the axis if revolution is shown by a dotted line and marked "P". The curve shown joining the two ends of this line has been rotated about the line "P" to form the header cap of the invention. Figure 4 is a cross section through said header cap (with an additional base section, discussed above). The outlet in this embodiment is in the plane of revolution, and preferably the full width of the hollow section (the full width of line "P").

In a preferred embodiment, the header cap of the invention comprises a curved surface. Preferably the curved surface, forming a hollow. The open section of said curved hollow comprises the outlet port. The outlet port may comprise the entire width of the hollow, or a partial width of the hollow form. The inlet port is attached to the curved surface, preferably essentially orthogonal to the outlet port. In a preferred embodiment, the inlet port is located on the curved surface at or around the most distal point from the outlet port.

In one embodiment, the dialyzer header cap of the invention improves uniformity of blood distribution to the hollow fibres of the dialyzer. This means that, rather than the majority of the blood flowing quickly through the centre of the

In one embodiment, the geometry of the dialyzer header cap is configured to reduce mechanical stress to blood flow within it. This occurs because the fluid entering the header cap through the inlet port can immediately expand to fill the hollow space inside, rather than reaming under higher pressure as it travels along a long and narrow inlet port (as shown in **Figure 1)** Preferably mechanical stress is reduced by 5% or more, 10% or more, 20% or more or 50% or more. In a most preferable embodiment, mechanical stress is reduced by 12% or more.

Preferably mechanical stress on blood is reduced through a reduction in pressure as the blood enters the dialyzer through the wide, hollow header cap of **Figures 3-8,** rather than through the narrow input port of the header cap of **Figure 1.**

In one embodiment, the present invention includes a dialyzer header cap wherein blood transfer efficiency into the hollow fibres of the dialyzer is improved. This occurs because when the blood enters the hollow chamber of the header cap, it is able to flow to fill the fill width of the header cap, which corresponds to the width of the bundle of hollow fibres. In other words, increased blood transfer efficiency into the dialyzer is one result of increased blood flow distribution uniformity at the outlet of the header cap. Therefore, the blood can more easily fill all of the fibres, and at a more equal rate, rather than being forced toward the centre of the bundle quickly, as occurs in the dialyzer header cap of **Figure 1** [illustrated experimentally in Figure 9]

In view of the above, it is clear that in preferred embodiments, the dialyzer header cap described herein is configured to increase blood flow distribution uniformity at the outlet. Preferably, blood flow distribution is increased by at least 10%, at least 20 %, at least 30%, at least 40%. In preferred embodiments, blood flow distribution is increased by at least 30-35%, most preferably around 33%.

Improved transfer efficiency of any fluid, such as priming solution, into the hollow fibres of the dialyzer, will also occur using the header cap of the invention. This is for the same reasons as above. Wherein the transferred fluid is blood, improved transfer efficiency will also occur due to the reduced mechanical stress (discussed above) leading to reduced clotting and damage to blood cells and hence the liquid being less viscous.

In one embodiment, the present invention includes a dialyzer header cap wherein urea mass transfer efficiency is increased. During dialysis, osmosis carries waste products from the blood inside the dialyzer hollow fibres into the dialysate surrounding the hollow fibres. Preferably urea is removed from the blood this way. Urea mass transfer efficiency is a way of measuring and monitoring the efficiency and effectiveness of the any type of dialysis.

In a preferred embodiment of the current invention mass transfer efficiency of urea is increased by up to 5% of urea koA, preferably up to 10% of urea koA, more preferably up to 20% of urea koA, more preferably up to 30% of urea koA.

In one embodiment, the geometry of the dialyzer header cap is configured to reduce air bubble formation within the dialyzer specifically, and more generally within the entire dialysis flow path. Air bubble formation in fluid, particularly blood, is potentially harmful to patients and reduced efficiency of the dialysis flow path. Air bubbles may coagulate and form foam. Preferably said air bubble formation is reduced by at least 10%, preferably by at least 20 %, preferably by at least 30%, most preferably by at least 40% by number of bubbles or by volume. In a preferred embodiment, air bubble formation is reduced by 25-30%, most preferably by around 29-30% or around 29.5% by number of bubbles or by volume. Air bubble formation may be measured using CFD simulation, amongst other methods.

Also herein is described a dialyzer including the dialyzer header cap described above. Said dialyzer preferably comprises at least the dialyzer header cap, a main housing onto which the dialyzer header cap can connect or attach at one end, and a bundle of hollow fibres.

## Kits

The dialyzer header cap disclosed herein can be provided in a kit.

Said kit may comprise one or more dialyzers.

Said kit may comprise a main housing for a dialyzer.

Said kit may comprise a bundle of hollow fibres, preferably said hollow fibres being housed inside a main housing.

Said kit may comprise connecting tubing, pipes or other vessels.

Said kit may comprise one or more sorbet cartridges.

Said kit may comprise connectors. Preferably the components of said kit can be connected to a water source and a dialysate source.

Said kit may comprise a dialysate or the components thereof.

A kit of the invention may include a coating to apply, or already applied to the dialyzer header cap and/or any other components of the kit. Said coating may comprise an antimicrobial agent, an antibacterial agent, a disinfectant, a lubricant, an anti-coagulant, an anti-viral agent or any combination of these. The coating may be applied totally or partially to the dialyzer header cap or any other kit components.

**Methods**

Also described herein are methods of use of the dialyzer header cap disclosed.

The dialyzer header cap may be used for priming. Priming is usually carried out for an entire dialysis flow path, including one or more dialyzer and a dialysis machine.

The steps for priming include firstly connecting the outlet of a dialysis machine to a sterile priming solution, preferably a sterile saline solution, and secondly operating the dialysis machine in reverse, in order to fill a dialysis flow path with saline. During this process the priming solution passes through a dialyzer with the header cap of the invention attached. The priming solution may then be optionally discarded since it may contain significantly high amounts of sterilant which accumulates if the flow path, and in particular a dialyzer in the flow path, has been previously sterilised and reused following a previous dialysis treatment.

If the priming solution is discarded, the first and second priming steps discussed above are repeated. For a third step, the inflow and outflow points of the flow path are then connected, and the priming solution circulated by the dialysis machine in forward operation.

The purpose of priming is to remove air and sterilant from the dialysis flow path, including the dialyzer. If air bubbles were to enter the arterial or venous bloodstream, the patient's pulmonary blood flow could be blocked, causing potentially serious complications, most commonly an air embolism.

The header cap of the current invention reduces air bubble formation in a dialysis flow path during priming. Preferably air bubble formation is reduced by at least 10% by volume, preferably by at least 20% by volume, preferably by at least 30% by volume, most preferably by at least 40% by volume. Preferably air bubble formation is reduced by at least 10% by number of bubbles, preferably by at least 20% by number, preferably by at least 30% by number, most preferably by at least 40% by number.

Sterilants are designed to destroy pathogens that may be present in the dialyzer and other parts of a dialysis flow path. Such products can be harmful to humans, and may possibly cause blood poisoning if they are allowed to enter the patient's blood stream. Priming removes sterilants from a dialysis flow path.

Another advantage of priming is that it warms the saline so the patient does not get too cool when dialysis treatment begins.

The dialyzer header cap may be used in method of hemodialysis.

**Uses**

The dialyzer header cap of the invention may be used in dialysis, particularly hemodialysis peritoneal dialysis, hemofiltration, hemodiafiltration or ultrafiltration.

The dialyzer header cap of the invention may also be used in priming a dialysis machine and a dialysis flow path comprising said dialysis machine, as described above.

Use of the header cap of the invention may also occur in the process of sterilizing a dialysis machine and all associated equipment in a dialysis flow path. During such a use, a sterliant passes through at least one dialyzer, including the header cap of the invention attached to said dialyzer.

***In Silico* Analysis**

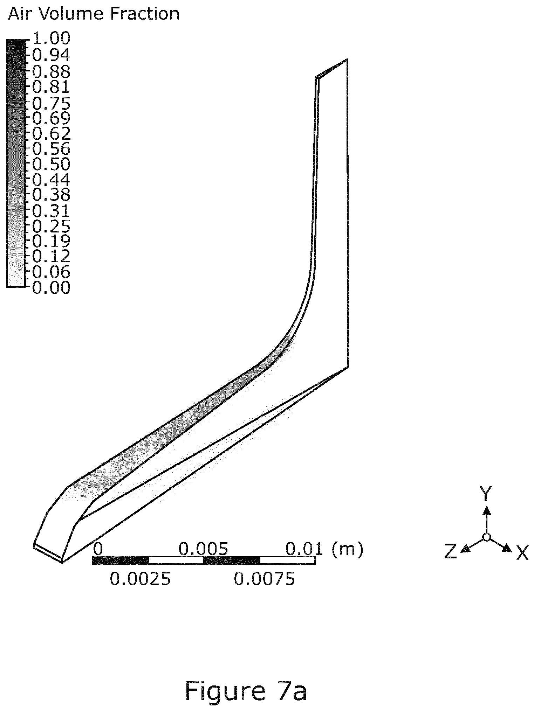

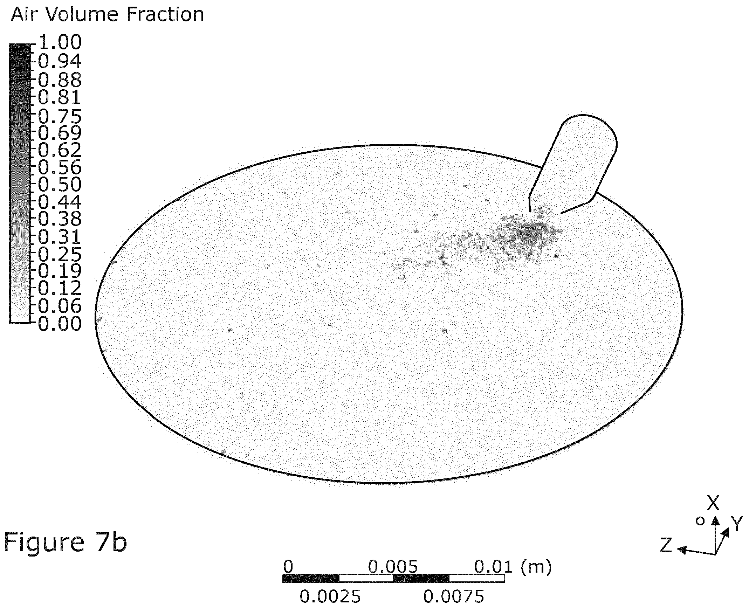

Numerical analysis of the dialyzer header port of the invention is shown in **Figures 6 and 7.** The blood flow velocity of the header cap of the current invention was measured using a blood fluid model and this was compared to the blood flow velocity of the existing header cap of **Figure 1.** The numerical data in **Figure 6** shows that the blood flow velocity was much greater towards a single central point for the header cap of Figure 1. For the header cap of the current invention, blood flow velocity was slower and evenly distributed across the entire width of the dialyzer. In other words, blood flow distribution uniformity is increased for the header caps of the current invention.

The trapped air volume during the priming phase in a dialysis flow path using a dialyzer header cap of the prior art **(Figure 7a)** was measured and compared to a header cap of the present invention **(Figure 7b).** The dialyzer header cap of the current invention is shown numerically to reduce the air volume fraction (the air volume to total volume ratio inside the header cap) during priming by 29.5% compared to the header cap shown in **Figure 1.** Therefore, the advantages of the dialyzer header cap described within have been demonstrated numerically.

**Claims**

1. A dialyzer header cap, comprising an curved surface forming a hollow, wherein said hollow includes an opening which comprises an outlet port, and further comprising an inlet port attached to the curved surface, wherein the inlet port is orthogonal to the outlet port, wherein said outlet port is in a plane of revolution and the full width of the hollow, and wherein said hollow forms a hollow halved solid of revolution created by rotating a plane curve 180° around an axis of revolution that lies on the same plane of revolution and where said plane curve is asymmetrical, wherein the said dialyzer header being **characterized in that** it is a hollow half a solid of revolution having a lachrymiform, tear drop shape.

2. A dialyzer header cap according to claim 1 wherein the inlet port is located on the curved surface at or around the most distal point from the outlet port.

3. A dialyzer header cap according to claim 1 wherein the outlet port of the header cap is formed along the axis of revolution, and wherein the inlet port is attached on or around the most distal point of the header cap from the outlet port.

4. A dialyzer header cap according to any preceding claim, wherein the header cap has an asymmetrical concave shape.

5. A dialyzer header cap according to claim 4, wherein the header cap has an internal volume generated by the cross-section of the outlet along a rotated axial direction less than about 40 degrees, less than about 35 degrees or less than about 30 degrees.

6. A dialyzer header cap according to any preceding claim, wherein said cap is formed of a plastic, a rubber, or a composite, optionally wherein said cap comprises of a plastic polycarbonate or polypropylene material.

7. A dialyzer header cap according to any preceding claim, wherein said cap comprises a plug fit, screw fit, stretch fit, Luer-Lock or snap fit attachment at the outlet port, inlet port or both.

8. A dialyzer comprising the dialyzer header cap according to any preceding claim.

9. A dialysis kit comprising a dialyzer header cap according to any of claims 1 to 7, a main housing and a bundle of hollow fibres inside the housing.

10. Use of a dialyzer header cap according to any of claims 1-7 in priming a dialyzer.

11. A method of priming a dialyzer according to claim 8, comprising the steps of

(a) connecting the outlet port of a dialysis machine in a dialysis flow path, including a dialyzer, to a sterile priming solution;
(b) operating the dialysis machine in reverse, in order to fill the flow path with saline;
(c) optionally discarding the priming solution if it contains or may contain significantly high amounts of sterilant;
(d) repeating steps (a) and (b); and
(e) connecting the inflow and outflow points of the dialysis flow path, and the circulating the priming solution by the dialysis machine in forward operation.

**Patentansprüche**

1. Dialysatorkopfkappe, umfassend eine gekrümmte Oberfläche, die einen Hohlraum ausbildet, wobei der Hohlraum eine Öffnung einschließt, die einen Auslassanschluss umfasst, und ferner umfassend einen Einlassanschluss, der an der gekrümmten Oberfläche befestigt ist, wobei der Einlassanschluss orthogonal zu dem Auslassanschluss ist, wobei der Auslassanschluss sich in einer Rotationsebene und über der gesamten Breite des Hohlraums befindet, und wobei der Hohlraum einen hohlen halbierten Rotationsfestkörper ausbildet, der durch Drehen einer Ebenenkurve um 180° um eine Rotationsachse erstellt wird, die auf derselben Rotationsebene liegt, und wobei die Ebenenkurve asymmetrisch ist, wobei der Dialysatorkopf **dadurch gekennzeichnet ist, dass** es sich um eine hohle Hälfte eines Rotationsfestkörpers handelt, der eine tränenförmige Tropfenform aufweist.

2. Dialysatorkopfkappe nach Anspruch 1, wobei der Einlassanschluss sich auf der gekrümmten Oberfläche an oder um den distalsten Punkt von dem Auslassanschluss befindet.

3. Dialysatorkopfkappe nach Anspruch 1, wobei der Auslassanschluss der Kopfkappe entlang der Rotationsachse ausgebildet ist und wobei der Einlassanschluss an oder um den distalsten Punkt der Kopfkappe von dem Auslassanschluss befestigt ist.

4. Dialysatorkopfkappe nach einem der vorstehenden Ansprüche, wobei die Kopfkappe eine asymmetrisch konkave Form aufweist.

5. Dialysatorkopfkappe nach Anspruch 4, wobei die Kopfkappe ein Innenvolumen aufweist, das durch den Querschnitt des Auslasses entlang einer gedrehten axialen Richtung von weniger als etwa 40 Grad, weniger als etwa 35 Grad oder weniger als etwa 30 Grad erzeugt wird.

6. Dialysatorkopfkappe nach einem der vorstehenden Ansprüche, wobei die Kappe aus einem Kunststoff, einem Gummi oder einem Verbundwerkstoff ausgebildet ist, optional wobei die Kappe aus einem Kunststoffpolycarbonat- oder Polypropylenmaterial besteht.

7. Dialysatorkopfkappe nach einem der vorstehenden Ansprüche, wobei die Kappe eine Steckpassung, Schraubpassung, Dehnpassung, Luer-Lock- oder Schnappverbindungsbefestigung an dem Auslassanschluss, dem Einlassanschluss oder beiden umfasst.

8. Dialysator, umfassend die Dialysatorkopfkappe nach einem der vorstehenden Ansprüche.

9. Dialysekit, umfassend eine Dialysatorkopfkappe nach einem der Ansprüche 1 bis 7, ein Hauptgehäuse und ein Bündel von Hohlfasern innerhalb des Gehäuses.

10. Verwendung einer Dialysatorkopfkappe nach einem der Ansprüche 1 bis 7 bei einem Priming eines Dialysators.

11. Verfahren für das Priming eines Dialysators nach Anspruch 8, umfassend die Schritte:

   (a) Verbinden des Auslassanschlusses einer Dialysemaschine in einem Dialysefließpfad, einschließlich eines Dialysators, mit einer sterilen Priming-Lösung;
   (b) umgekehrtes Betreiben der Dialysemaschine, um den Fließpfad mit Kochsalzlösung zu füllen;
   (c) optional, Entsorgen der Priming-Lösung, wenn sie wesentlich hohe Mengen an Sterilisationsmittel enthält oder enthalten könnte;
   (d) Wiederholen der Schritte (a) und (b); und
   (e) Verbinden der Zufluss- und Abflusspunkte des Dialysefließpfads und das Zirkulieren der Priming-Lösung durch die Dialysemaschine in einem Vorwärtsbetrieb.

**Revendications**

1. Capuchon de collecteur de dialyseur, comprenant une surface incurvée formant un creux, dans lequel ledit creux comporte une ouverture qui comprend un orifice de sortie, et comprenant en outre un orifice d'entrée fixé à la surface incurvée, dans lequel l'orifice d'entrée est orthogonal à l'orifice de sortie, dans lequel ledit orifice de sortie se trouve dans un plan de révolution et toute la largeur du creux, et dans lequel ledit creux forme un demi-solide creux de révolution créé par la rotation d'une courbe plane de 180° autour d'un axe de révolution qui se trouve sur le même plan de révolution et où ladite courbe plane est asymétrique, dans lequel ledit collecteur de dialyseur est **caractérisé en ce qu'il** est un demi-solide creux de révolution ayant une forme de goutte d'eau lachrymiforme.

2. Capuchon de collecteur de dialyseur selon la revendication 1, dans lequel l'orifice d'entrée est situé sur la surface incurvée au niveau du point le plus distal de l'orifice de sortie ou autour de celui-ci.

3. Capuchon de collecteur de dialyseur selon la revendication 1, dans lequel l'orifice de sortie du capuchon de collecteur est formé le long de l'axe de révolution, et dans lequel l'orifice d'entrée est fixé sur le point le plus distal du capuchon de collecteur ou autour de celui-ci par rapport à l'orifice de sortie.

4. Capuchon de collecteur de dialyseur selon l'une quelconque revendication précédente, dans lequel le capuchon de collecteur a une forme concave asymétrique.

5. Capuchon de collecteur de dialyseur selon la revendication 4, dans lequel le capuchon de collecteur a un volume interne généré par la section transversale de la sortie le long d'une direction axiale de rotation inférieure à environ 40 degrés, inférieure à environ 35 degrés ou inférieure à environ 30 degrés.

6. Capuchon de collecteur de dialyseur selon l'une quelconque revendication précédente, dans lequel ledit capuchon est formé d'un plastique, d'un caoutchouc ou d'un composite, éventuellement dans lequel ledit capuchon est constitué d'un matériau plastique en polycarbonate ou en polypropylène.

7. Capuchon de collecteur de dialyseur selon l'une quelconque revendication précédente, dans lequel ledit capuchon comprend un raccord bouchon, un raccord à vis, un raccord extensible, un raccord Luer-Lock ou par encliquetage au niveau de l'orifice de sortie, de l'orifice d'entrée ou des deux.

8. Dialyseur comprenant le capuchon de collecteur de dialyseur selon l'une quelconque revendication précédente.

9. Kit de dialyse comprenant un capuchon de collecteur de dialyseur selon l'une quelconque des revendications 1 à 7, un boîtier principal et un faisceau de fibres creuses à l'intérieur du boîtier.

10. Utilisation d'un capuchon de collecteur de dialyseur selon l'une quelconque des revendications 1-7 pour l'amorçage d'un dialyseur.

11. Procédé d'amorçage d'un dialyseur selon la revendication 8, comprenant les étapes consistant à

(a) connecter l'orifice de sortie d'une machine de dialyse dans une voie d'écoulement de la dialyse, y compris un dialyseur, à une solution d'amorçage stérile ;
(b) faire fonctionner la machine de dialyse en sens inverse, afin de remplir la voie d'écoulement avec du sérum physiologique ;
(c) éventuellement, jeter la solution d'amorçage si elle contient ou peut contenir des quantités significativement élevées d'agents stérilisants ;
(d) répéter les étapes (a) et (b) ; et
(e) connecter des points d'entrée et de sortie de la voie d'écoulement de la dialyse, et la circulation de la solution d'amorçage par la machine de dialyse en marche avant.

FIGURE 3

3a

BLOOD INFLOW

BLOOD OUTFLOW

B

3b

BLOOD INFLOW

BLOOD
OUTFLOW

A

A

B

3c

BLOOD INFLOW

Φ4

BLOOD OUTFLOW

BACK Ø16

Ø54,90

Figure 3d

RIGHT

19,93

23,64

Figure 3e

SECTION A-A
SCALE 4:1

Figure 4

EP 3 900 814 B1

SECTION B-B
SCALE 2:1

(40,15)

6,93

Figure 5

19

Axial velocity on normalized diameter position - Inflow Blood Port (Qb=300 mL/min)

- - original shape    --- asymmetrical concave shape

Figure 6a

Figure 6b

Air Volume Fraction

Figure 7a

Figure 7b

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4141835 A

**Non-patent literature cited in the description**

- **KIM et al.** Effects of Arterial Port Design on Blood Flow Distribution in Hemodialyzers. *Blood Purification*, 2009, vol. 28 (3), 260-7

- **KULZ et al.** In vitro and in vivo evaluation of a new dialyzer. *Nephrol. Dial. Transplant.*, 2002, vol. 17 (8), 1475-9